**Europäisches Patentamt**

**European Patent Office**

**Office européen des brevets**

(19)

(11) Numéro de publication: **0 038 759 B1**

(12) **FASCICULE DE BREVET EUROPEEN**

(45) Date de publication du fascicule du brevet: 07.03.84

(51) Int. Cl.³: **C 12 M 1/00**

(21) Numéro de dépôt: **81400622.7**

(22) Date de dépôt: **17.04.81**

(54) **Installation pour la préparation des gaz combustibles par fermentation.**

(30) Priorité: **21.04.80 FR 8008855**

(43) Date de publication de la demande: **28.10.81 Bulletin 81/43**

(45) Mention de la délivrance du brevet: **07.03.84 Bulletin 84/10**

(84) Etats contractants désignés: **AT BE CH DE GB IT LI LU NL SE**

(56) Documents cités:
**BE - A - 507 640**
**FR - A - 898 669**
**FR - A - 914 808**
**FR - A - 1 011 722**

(73) Titulaire: **Dedenon, Jean-Marie, 10, Rue du 8 Mai, F-77370 Nangis (FR)**
Titulaire: **Rassak, Denis, 15, Rue Georges Blandon, F-78430 Louveciennes (FR)**

(72) Inventeur: **Dedenon, Jean-Marie, 10, Rue du 8 Mai, F-77370 Nangis (FR)**
Inventeur: **Rassak, Denis, 15, Rue Georges Blandon, F-78430 Louveciennes (FR)**

(74) Mandataire: **Ores, Irène et al, CABINET ORES 6, Avenue de Messine, F-75008 - Paris (FR)**

ACTORUM AG

Installation pour la préparation des gaz combustibles par fermentation

La présente invention est relative à des perfectionnements apportés aux installations et aux procédés de préparation des gaz combustibles par fermentation. Elle est plus particulièrement relative à des fermentations méthanigènes anaérobies ainsi qu'aux installations pour la mise en œuvre de ces fermentations.

Il est connu de préparer les gaz combustibles, et notamment le méthane, par fermentation anaérobie de masses constituées par des déchets organiques agricoles, urbains ou même industriels, s'ils contiennent suffisamment de matières organiques. Pour que la production des gaz soit ininterrompue et le prix de revient compétitif, généralement les méthodes de travail appliquées doivent être des méthodes de travail en continu. Un processus continu présente encore d'autres très grands avantages comme, par exemple, réduction des temps morts nécessaires au nettoyage, au remplissage, à la vidange, etc., diminution des volumes de l'ensemble de l'appareillage, grande facilité de travail, possibilité de mécanisation et d'automatisation des opérations, augmentation du rendement dû à un meilleur choix des conditions opératoires.

De nombreuses installations de fermentation méthanigènes ont été décrites, et cela d'autant plus que les besoins accrus en énergie et la raréfaction des sources d'énergie classique à base de pétrole poussent les chercheurs et les industriels à utiliser cette matière première abondante et bon marché que constitue la biomasse. Ainsi:

— le brevet français N° 79.05662 décrit un procédé de préparation du méthane à partir des fumiers de vache, par fermentation anaérobie, procédé qui se caractérise en ce que le substrat est introduit dans une première chambre de traitement préalable où il est porté à une température de l'ordre de 70°C, puis transporté à une température d'environ 55-60°C dans une deuxième chambre de traitement et mis en contact avec une culture de micro-organismes;

— le brevet français N° 77.35966 décrit une installation comportant une batterie de trois cuves disposées les unes au-dessus des autres, la fermentation étant aérobie dans la première et anaérobie dans les deux autres;

— le brevet français N° 77.22386 décrit un procédé de production de méthane par fermentation anaérobie des déchets organiques dans une cuve de fermentation étanche et complètement remplie d'eau;

— le brevet français N° 75.09057 décrit une installation de digestion des matières organiques par fermentation anaérobie, comportant une enceinte à deux compartiments communiquant par leurs parties supérieures;

— le brevet allemand N° 2535756 décrit une installation de production de méthane qui consiste en une cuve cylindrique inclinée sur l'horizontale et munie d'un agitateur axial;

— le brevet américain N° 4022665 décrit un procédé de digestion anaérobie des déchets organiques pour la production de méthane, procédé qui comporte deux étapes distinctes, une première étape de production d'acides organiques de bas poids moléculaire, et une deuxième étape de transformation de ces composés en méthane;

— le brevet français N° 914808 décrit un digesteur pour ordures de villes, dans lequel le liquide est introduit latéralement en dessous de la trémie;

— le brevet français N° 898669 décrit un appareillage et un procédé de digestion avec production de gaz combustibles, appareillage assez simple d'ailleurs, ne comportant aucun panier ni aucun dispositif de décharge.

D'autres brevets et publications encore concernent également la préparation du méthane par fermentation anaérobie.

En effet, la digestion de déchets organiques dans un processus anaérobie est connue depuis des dizaines d'années. Un tel processus utilise des cultures microbiennes mixtes et divers substrats. De très nombreux micro-organismes sont capables de produire du méthane et ils sont présents universellement dans la nature (marais, fumier, rouissage du lin, décomposition de la matière organique dans le sol, dégradation de la cellulose, etc.). Toutefois, on n'a pas pu mettre encore au point une installation économiquement rentable, très robuste et facile à conduire et à mettre en œuvre dans toutes les fermes, principaux fournisseurs de la matière première telle que fumier, purin, paille, etc. Les raisons en sont fort simples, celles-là mêmes qui expliquent pourquoi les solutions préconisées dans les brevets cités ci-dessus ne sont pas non plus économiquement viables. Ce sont principalement les suivantes:

Les installations de fermentation, et notamment les fermentations continues, connues jusqu'à présent opèrent en milieu aqueux, dilué. Ces fermentations sont à rapprocher des digestions et traitements des eaux d'égouts, dont la charge optimale se situe autour de 8% de matière organique. Dans les installations de fermentation continue, un agitateur ou un dispositif analogue est nécessaire pour briser la croûte qui se forme à la surface libre, qui sert de surface de dégagement de gaz produits (le maintien de celle-ci est dangereux pour l'installation). Le transfert des fluides et le brassage requièrent des pompes puissantes, consommatrices d'énergie. La réaction globale étant en outre très peu exothermique, le réchauffage et le maintien de la température nécessaire dans les zones de fermentation mézophiles ou thermophiles de la très grande masse d'eau présente consomment également beaucoup d'énergie. A titre d'exemple, on estime que le traitement d'une boue à 2% de matière fermentescible consomme à peu près toute la production de méthane qui sert à chauffer et à maintenir la température nécessaire au fonctionnement du digesteur. De plus, dans la plupart de ces installations continues, le résidu de la fermentation lui-même est également trop aqueux pour une utilisation directe comme engrais organique.

La présente invention s'est par conséquent donné pour but de fournir un procédé et une installation de préparation de gaz combustibles par fermentation anaérobie des déchets organiques, qui répondent mieux aux nécessités de la pratique que les procédés et les installations visant au même but antérieurement connus, notamment en ce qu'ils permettent une très importante économie d'énergie (absence d'agitation et déroulement de la fermentation en milieu plus concentré); en ce qu'ils évitent la formation des croûtes à la surface, très dangereuses surtout pour les fermentations méthanigènes; en ce qu'ils permettent un déroulement mécanisé et quasi automatique des opérations; en ce qu'ils sont d'un maniement très facile; en ce qu'ils sont très souples et permettent de traiter aussi bien des milieux solides avec des taux d'humidité variables, tels que fumiers, pailles, feuilles, fanes, ordures ménagères, etc., que des milieux liquides, solutions et suspensions aqueuses de divers types, tels que purins, lisiers, urines, eaux-vannes, aussi bien d'une manière continue que semi-continue ou même discontinue, et en ce qu'ils fournissent, d'une part, un résidu solide de fermentation très peu aqueux et facilement transportable et pouvant servir d'engrais et, d'autre part, un liquide clair sans suspension aucune, pratiquement non polluant, facilement rejetable dans les égouts et les rivières.

La présente invention a pour objet une installation pour la préparation des gaz combustibles par fermentation anaérobie, caractérisée en ce qu'elle comporte en combinaison: au moins une cuve pour le traitement de solides ou de substrats très concentrés (présentant une teneur supérieure à 16% de matières sèches), ladite cuve étant munie de cloisons perforées assurant un drainage sensiblement sur toute la hauteur de la cuve, maintenant les solides et laissant circuler les liquides et les gaz; au moins une cuve pour le traitement de substrats dilués (dont la teneur en matières sèches est inférieure à 10%) équipée d'un serpentin de chauffage; un dispositif d'introduction de substrats solides; un appareil pour l'introduction de substrats liquides; un dispositif de récolte de résidu de fermentation provenant de la cuve ou des cuves de fermentation de substrat solide; des tubulures de liaison entre les cuves de fermentation de substrat solide et les cuves de fermentation de substrat liquide, lesdites tubulures reliant les cuves de fermentation à des niveaux différents; un tube collecteur des gaz produits par fermentation.

Conformément à l'invention, le dispositif d'introduction de matières solides est pourvu d'un hachoir, d'un tube d'arrivée de jus de recirculation de la cuve de fermentation du substrat liquide et d'un organe d'entraînement comportant une hélice d'entraînement ou analogue tel que pompe, agitateur, terminé ou non par un organe de répartition.

Suivant une modalité particulièrement avantageuse de ce mode de réalisation, les différents éléments formant le dispositif d'introduction des matières solides sont disposés de façon à former deux joints hydrauliques successifs antérieur et postérieur entre l'atmosphère extérieure et le volume interne de la cuve de fermentation du substrat solide.

Ce dispositif qui hache, fluidise et répartit convenablement le substrat solide qui alimente la cuve de fermentation dudit substrat permet non seulement d'avoir une composition et une granulométrie homogènes et une biomasse qui descend facilement par gravité, mais également d'obtenir un résidu de fermentation homogène fournissant un engrais facile à utiliser.

Selon un mode de réalisation avantageux de l'objet de l'invention, la cloison perforée dont est pourvue la cuve de fermentation du substrat solide a la forme d'un panier présentant la forme de ladite cuve.

Ce panier est placé à quelque distance de la paroi de la cuve, juste pour laisser passer le liquide et le gaz, les solides étant retenus à l'intérieur.

Suivant un autre mode de réalisation de l'objet de l'invention, la cloison perforée dont est pourvue la cuve de fermentation du substrat solide est disposée de manière à séparer la cuve en plusieurs zones contenant le substrat solide et plusieurs zones d'écoulement des liquides et des gaz.

Conformément à l'invention, le dispositif de récolte du résidu de fermentation provenant de la cuve de fermentation du substrat solide se compose d'un jeu de grilles équipées d'un mécanisme d'arrachage du substrat fermenté facilement manœuvrable, et d'une trémie d'évacuation pourvue d'un organe d'extraction constituant un joint hydraulique d'isolement du volume intérieur de la cuve.

Selon un mode de réalisation particulièrement avantageux de l'objet de l'invention, le fermenteur du substrat solide est situé au-dessus de la cuve de fermentation du substrat liquide et/ou en solution afin de permettre l'écoulement par gravité du jus provenant du fermenteur supérieur vers le fermenteur inférieur.

Conformément à l'invention, le gaz produit dans les fermenteurs est récolté directement par les tubulures et conduites partant des fermenteurs, tandis qu'une certaine quantité de ce gaz passe, avant d'être récoltée, à travers le joint hydraulique postérieur du dispositif d'alimentation en substrat solide.

Cette disposition, bien qu'elle contamine légèrement en air le méthane récolté, permet, par contre, de travailler dans une atmosphère parfaitement anaérobie.

La présente invention a également pour objet un procédé de préparation des gaz combustibles, par fermentation anaérobie, caractérisé en ce que l'on charge à l'abri de l'air un substrat solide dans la cuve de fermentation du substrat solide déjà éventuellement particulièrement remplie de substrat préalablement introduit par la même voie et en cours de fermentation, en ce que l'on hache éventuellement ledit substrat solide à l'aide d'un hachoir présent dans le dispositif d'alimentation, l'introduction étant, le cas échéant, facilitée par l'admission, dans le dispositif d'alimentation, du jus de fermentation provenant d'une cuve de fermentation des substrats liquides, en ce que les couches inférieures du substrat solide sont évacuées au fur et à mesure à l'aide d'un dispositif de récolte de résidu de fermentation, la fréquence et les débits d'alimentation et d'évacuation étant coordonnés de manière que le substrat séjourne dans la cuve de fermentation entre environ 1 et 60 d, en ce que le jus liquide s'écoule dans la cuve de fermentation des substrats liquides située généralement au-dessous de la cuve de fermentation du substrat solide, tandis que le gaz produit est récolté par l'intermédiaire de tubulures prévues à cet effet, et en ce que, d'autre part, le substrat liquide et/ou en solution est chargé à l'abri de l'air dans une cuve de fermentation du substrat liquide, cuve munie d'un serpentin pour le chauffage des liquides, de moyens de recirculation du jus vers la cuve de fermentation du substrat solide et de tubulures de récoltes du gaz produit.

Ainsi, chaque fois que l'on introduit une nouvelle couche de substrat solide, on peut évacuer une couche du substrat digéré, le rythme et la durée de séjour étant fonction de la qualité du substrat de départ et de la qualité désirée du substrat digéré, c'est-à-dire d'engrais.

Le jus de recirculation provenant du digesteur liquide remplit plusieurs fonctions: en s'écoulant du et à travers le substrat solide contenu dans la cuve à substrat solide qui constitue avec son système de cloisons perforées une sorte de percolateur, il se débarrasse des particules solides en suspension et, ainsi, on clarifie le jus qu'il est ensuite possible de déverser sans inconvénient; d'autre part, étant réchauffé par suite de la présence d'un serpentin de chauffage dans la cuve à substrat liquide, il permet, si on le désire, de réchauffer et de maintenir la biomasse présente dans la ou les cuves à substrat solide à la température désirée.

La recirculation de ce jus permet encore:
— de contrôler l'activité du milieu du fermenteur à substrat solide;
— d'ajuster le degré d'humidité de la biomasse;
— de disposer d'une sorte de pied de cuve permanent favorisant un démarrage rapide de la fermentation des matières nouvellement introduites.

Outre les dispositions qui précèdent, l'invention comprend encore d'autres dispositions, qui ressortiront de la description qui va suivre.

La présente invention vise plus particulièrement les procédés et les installations de production du gaz combustible conformes aux dispositions qui précèdent, ainsi que les moyens propres à la mise en œuvre de ces procédés et à la réalisation de ces installations, de même que les procédés d'ensemble et les chaînes de fabrication dans lesquels sont inclus les procédés et les installations conformes à la présente invention.

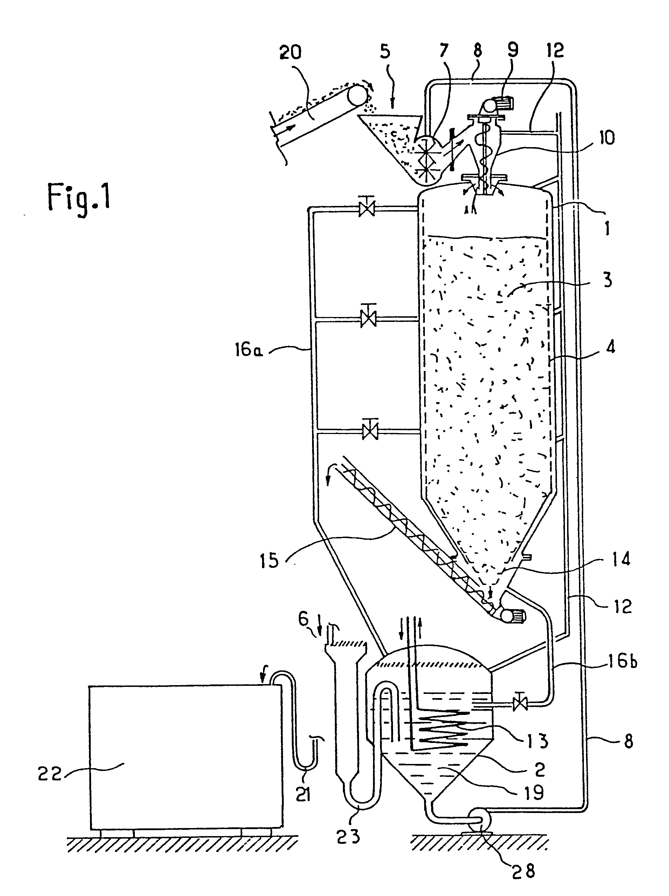

L'invention pourra être mieux comprise à l'aide du complément de description qui va suivre, qui se réfère aux dessins annexés dans lesquels les fig. 1 à 3 représentent schématiquement l'installation qui fait l'objet de la présente invention.

Il doit être bien entendu, toutefois, que l'installation décrite dans ce qui va suivre et représentée aux dessins est donnée uniquement à titre d'illustration de l'objet de l'invention, mais n'en constitue en aucune manière une limitation.

L'installation conforme à la présente invention qui produit du méthane par fermentation anaérobie et qui est représentée schématiquement à la fig. 1 comprend, non limitativement, une cuve cylindrique supérieure 1 au sommet de laquelle est placé l'organe de répartition 11 terminant le dispositif d'alimentation en substrat solide 5 (alimenté par exemple par le tapis roulant 20). Le dispositif d'alimentation 5 est muni d'un hachoir 7. Ainsi, le substrat solide 3, avant de pénétrer dans la cuve 1, est haché en menus morceaux par le hachoir 7 et est arrosé par le jus provenant du fermenteur liquide 2 arrivant par la conduite 8. Le mélange obtenu est entraîné par un organe mobile équipé d'une hélice 24 mue par un moteur 9 tel que vis d'Archimède, pompe ou analogue. Le panier perforé 4 maintient la biomasse solide 3, tandis que le jus s'écoule par gravité par la tuyauterie 16a, 16b, et que le gaz produit est récolté par l'intermédiaire des tuyauteries 12. Après la fermentation et le séjour plus ou moins long dans la cuve 1, le substrat solide 3 utilisé comme engrais est évacué de l'enceinte 1 par ouverture des grilles manœuvrables à fonctionnement mécanisé situées en bas de la cuve 1. L'évacuation peut avoir lieu, par exemple, à l'aide de la trémie munie de la vis d'évacuation 15. La cuve de digestion du substrat liquide 2 se situe par exemple au-dessous de la cuve 1 et reçoit par gravité le jus en provenance de la cuve 1. Elle est alimentée directement par l'intermédiaire de l'appareil 6 d'alimentation en substrat liquide 19 et elle est munie d'un serpentin 13 à circulation d'eau chaude, par exemple, pour un réchauffage éventuel du substrat liquide 19. Le jus épuisé (et clair) est envoyé, par l'intermédiaire de la conduite 21 (qui peut se brancher sur la conduite 16b, en bas de la cuve 1), dans le réservoir 22.

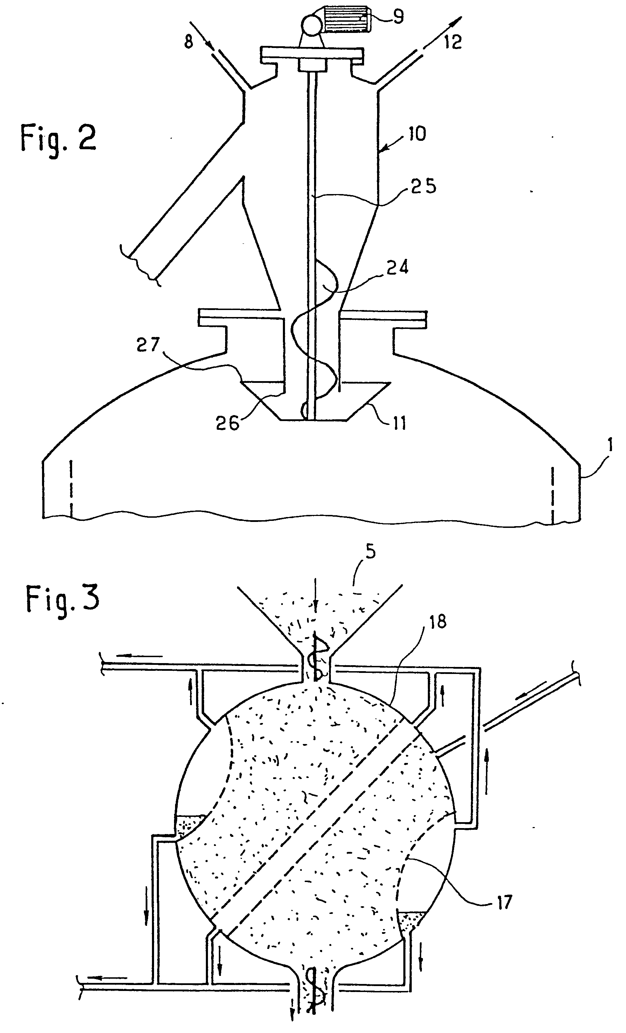

La fig. 2 représente en détail l'organe d'entraînement du dispositif d'alimentation en substrat solide 5. Le substrat haché et arrosé par le jus en provenance de la cuve 2 et arrivant par la conduite 8 (placée, ici, après le hachoir) est entraîné dans la cuve de fermentation 1 à l'aide d'un introducteur 10 dont l'axe 25 est muni à sa partie basse d'une hélice 24 d'entraînement des matières. Cet axe tourne, mû par le moteur 9. L'ensemble constitue un organe d'introduction forcée tel que vis d'Archimède, pompe ou analogue. Il peut être muni également, à sa partie inférieure, d'une soucoupe 11 montée de telle sorte que son bord supérieur 27 soit plus haut de quelques centimètres que le bas du tube d'alimentation 26 qui termine le corps de l'introducteur 10 du dispositif d'alimentation. Ce dispositif d'alimentation est également relié, par l'intermédiaire de la conduite 12, au réservoir de stockage du méthane.

La fig. 3 représente une autre variante d'une cuve de fermentation du substrat solide; la cuve présente la forme d'une enceinte sphérique 18 subdivisée en plusieurs parties à l'aide de cloisons perforées 17.

L'ensemble de l'opération pourrait, par exemple, se dérouler de la manière suivante: le tapis roulant 20 amène le fumier frais et le déverse dans le dispositif d'alimentation 5. Le fumier est alors haché par le hachoir 7 et copieusement arrosé par le jus en provenance de la cuve de fermentation du substrat liquide 2 et qui arrive par l'intermédiaire de la conduite 8. Le jus est porté à 40°C environ au contact du serpentin à eau chaude 13 présent dans la cuve 2. Le hachoir 7 ainsi que la soucoupe de répartition 11 sont munis l'un et l'autre d'un joint d'eau qui constitue les joints hydrauliques antérieur et postérieur dudit dispositif d'alimentation 5. De cette manière, il ne peut pas s'établir de communication directe entre l'intérieur de la cuve 1 et l'atmosphère extérieure; l'important débit de jus chaud s'écoulant par la conduite 8 contribue à l'introduction du fumier frais. Le fumier frais ainsi ensemencé et réchauffé se trouve étalé dans la partie haute de la partie supérieure de la cuve 1 qui joue le rôle d'un percolateur. Grâce à la présence du panier perforé 4, on réalise l'égouttage en continu du substrat solide ainsi que l'échappement du gaz méthane. L'enveloppe extérieure de la cuve 1 est de préférence calorifugée. Grâce aux conduites 16 (16a et 16b), le jus percolé est dirigé vers la cuve de fermentation du substrat liquide 2. Il s'y trouve mélangé, par exemple, à du purin frais introduit par l'appareil d'admission du substrat liquide 6, par l'intermédiaire d'un double siphon 23. Le serpentin de réchauffage 13 maintient la température constante (environ 40°C). La pompe à recirculation du jus chaud 28 assure le retour pour l'ensemencement des matières fraîches introduites dans la cuve 1, ainsi que pour le réchauffage de la cuve 1. En branchant sur la conduite 16b une tubulure de trop-plein (non représentée sur le dessin), que l'on raccorde à la conduite 21 d'évacuation du jus épuisé, et en arrêtant la recirculation du jus chaud, la cuve 2 se remplit complètement et le jus épuisé doit emprunter la conduite 21 et se déverser dans la cuve à jus épuisé 22; ce processus assure le transfert automatique du trop-plein de jus circulant vers la cuve 22 à jus épuisé. A la base de la cuve 1, le fumier épuisé est évacué par un dispositif de récolte constitué d'un jeu de grilles équipées d'un mécanisme d'arrachage 14 et de la vis 15. Les conduites à gaz 12 prélèvent le gaz brut produit par les deux cuves 1 et 2 pour le diriger vers le conditionnement et l'utilisation.

La marche de l'installation telle que décrite peut être entièrement automatique et ne nécessite que la régulation de la température du jus de recirculation dans la cuve 2 et du débit convenable de percolation.

Il résulte de la description qui précède que, quels que soient les modes adoptés de mise en œuvre, de réalisation et d'application, on obtient des procédés et installations qui présentent, par rapport aux procédés et aux installations visant au même but antérieurement connus, des avantages importants, dont certains ont été mentionnés dans ce qui précède, et notamment:

— l'avantage de pouvoir réaliser la fermentation de manière continue, automatique et avec un minimum de dépense énergétique;

— l'avantage de pouvoir optimiser au maximum le traitement des substrats composites par la séparation des substrats concentrés et solides et des substrats dilués;

— l'avantage d'aboutir à un résidu solide homogène du point de vue granulométrique, formant un engrais de qualité facilement transportable et un effluent liquide clair et non polluant;

— l'avantage d'une très grande simplicité d'exploitation, pouvant être réalisée sur mesure en fonction de la quantité et de la qualité des matières premières, sur les lieux d'exploitation.

Ainsi que cela ressort de ce qui précède, l'invention ne se limite nullement à ceux de ses modes de mise en œuvre, de réalisation et d'application qui viennent d'être décrits de façon plus explicite dans ce qui précède; elle en embrasse, au contraire, toutes les variantes qui peuvent venir à l'esprit du technicien en la matière, sans s'écarter du cadre ni de la portée de la présente invention.

## Revendications

1. Installation pour la préparation de gaz combustibles, par fermentation anaérobie, caractérisée en ce qu'elle comporte en combinaison: au moins une cuve (1) pour le traitement de solides ou de substrats très concentrés (présentant une teneur supérieure à 16% de matières sèches), ladite cuve étant munie de cloisons perforées (4, 17) assurant un drainage sensiblement sur toute la hauteur de la cuve, maintenant les solides et laissant circuler les liquides et les gaz; au moins une cuve (2) pour le traitement de substrats dilués (dont la teneur en matières sèches est inférieure à 10%) équipée d'un serpentin de chauffage (13); un dispositif d'introduction de substrats solides (5) pourvu d'un hachoir (7), d'un tube d'arrivée (8) de jus de recirculation de la cuve de fermentation du substrat liquide et d'un organe d'entraînement (25) comportant une hélice d'entraînement (24), pompe ou analogue, terminé éventuellement par un organe de répartition (11); un appareil pour l'introduction de substrats liquides (6); un dispositif de récolte (14, 15) de résidu de fermentation provenant de la cuve ou des cuves de fermentation de substrat solide; des tubulures de liaison (16a, 16b) entre les cuves de fermentation de substrat solide et les cuves de fermentation de substrat liquide, lesdites tubulures reliant les cuves de fermentation à des niveaux différents; un tube collecteur (12) des gaz produits par fermentation.

2. Installation selon la revendication 1, caractérisée en ce que les différents éléments formant le dispositif d'introduction des matières solides sont disposés de façon à former deux joints hydrauliques successifs antérieur et postérieur entre l'atmosphère extérieure et le volume interne de la cuve de fermentation du substrat solide.

3. Installation selon la revendication 1, caractérisée en ce que la cloison perforée dont est pourvue la cuve de fermentation du substrat solide a la forme d'un panier présentant la forme de ladite cuve.

4. Installation selon la revendication 1, caractérisée en ce que la cloison perforée dont est pourvue la cuve de fermentation du substrat solide est disposée de manière à séparer la cuve en plusieurs zones contenant le substrat solide et plusieurs zones d'écoulement des liquides et des gaz.

5. Installation selon l'une des revendications 1 à 4, caractérisée en ce que le dispositif de récolte du résidu de fermentation provenant de la cuve de fermentation du substrat solide se compose d'un jeu de grilles équipées d'un mécanisme d'arrachage du substrat fermenté facilement manœuvrable et d'une trémie d'évacuation (15) pourvue d'un organe d'extraction constituant un joint hydraulique d'isolement du volume intérieur de la cuve.

6. Installation selon l'une des revendications 1 à 5, caractérisée en ce que le fermenteur du substrat solide est situé au-dessus de la cuve de fermentation du substrat liquide et/ou en solution, afin de permettre l'écoulement par gravité du jus provenant du fermenteur supérieur vers le fermenteur inférieur.

7. Installation selon l'une des revendications 1 à 6, caractérisée en ce que le gaz produit dans les fermenteurs est récolté directement par les tubulures et conduites partant des fermenteurs, tandis qu'une certaine quantité de ce gaz passe, avant d'être récoltée, à travers le joint hydraulique postérieur du dispositif d'alimentation en substrat solide.

## Patentansprüche

1. Einrichtung zum Erzeugen von brennbaren Gasen durch anaerobe Fermentation, dadurch gekennzeichnet, dass sie in Kombination folgendes umfasst: wenigstens einen Behälter (1) für die Behandlung von Feststoffen oder von sehr konzentrierten Substraten (die einen Gehalt von Trockenstoffen von über 16% haben), wobei dieser Behälter mit perforierten Trennwänden (4, 17) versehen ist, die eine Entwässerung im wesentlichen über die gesamte Höhe des Behälters sicherstellen, die Feststoffe festhalten und die Flüssigkeiten sowie das Gas zirkulieren lassen; wenigstens einen Behälter (2) für Behandlung von verdünnten Substraten (deren Gehalt an Trockenstoffen unterhalb von 10% liegt), der mit einer Heizschlange (13) ausgerüstet ist; eine Einrichtung (5) zum Einführen von festen Substraten, die mit einer Schneide- bzw. Zerkleinerungseinrichtung (7) versehen ist, sowie mit einem Zuführungsrohr (8) für Rezirkulationsbrühe von dem Behälter zur Fermentation von flüssigem Substrat und mit einem Mitnahmeorgan (25), das eine Transportschnecke (24), eine Pumpe oder ähnliches umfasst und eventuell in einem Verteilungsorgan (11) endet; eine Einrichtung (6) zum Einführen von flüssigen Substraten; eine Einrichtung (14, 15) zum Ausstossen der Rückstände der Fermentation, die aus dem Behälter oder den Behältern für die Fermentation von festem Substrat stammen; Verbindungsrohrleitungen (16a, 16b) zwischen den Behältern zur Fermentation von festen Substraten und den Behältern zur Fermentation von flüssigem Substrat, wobei diese Rohrleitungen die Behälter zur Fermentation auf unterschiedlichen Niveaus verbinden und ein Sammelrohr (12) für Gase, welche durch Fermentation erzeugt worden sind.

2. Einrichtung nach Anspruch 1, dadurch gekennzeichnet, dass die unterschiedlichen Elemente, welche die Einrichtung zum Einführen von festen Materialen bzw. Feststoffen bilden, in der Weise vorgesehen sind, dass sie zwei aufeinanderfolgende vordere und hintere hydraulische Dichtungen, von denen eine eine vordere und eine eine hintere hydraulische Dichtung ist, zwischen der äusseren Atmosphäre und dem inneren Volumen des Behälters zur Fermentation von festem Substrat bilden.

3. Einrichtung nach Anspruch 1, dadurch gekennzeichnet, dass die perforierte Trennwand, die in dem Behälter zur Fermentation des festen Substrats vorgesehen ist, die Form eines Korbes hat, der die Form dieses Behälters aufweist.

4. Einrichtung nach Anspruch 1, dadurch gekennzeichnet, dass die perforierte Trennwand, mit welcher der Behälter zur Fermentation des festens Substrats versehen ist, in der Weise vorgesehen ist, dass sie den Behälter in mehrere Zonen auftrennt, welche das feste Substrat enthalten sowie in mehrere Zonen der Abführung der Flüssigkeiten und der Gase.

5. Einrichtung nach einem der Ansprüche 1 bis 4, dadurch gekennzeichnet, dass die Einrichtung zum Austossen der Rückstände der Fermentation, die von dem Behälter zur Fermentation des festen Substrats stammen aus einem Satz von Gittern besteht, die mit einem Mechanismus des Herunterreissens oder sonstigen Entnehmens des fermentierten Substrats versehen sind, der leicht betätigbar ist, sowie aus einem Entleerungstrichter (15), der mit einem Entnahmeorgan bzw. Abführungsorgan versehen ist und eine hydraulische Dichtung zum Isolieren des inneren Volumens des Behälters bildet.

6. Einrichtung nach einem der Ansprüche 1 bis 5, dadurch gekennzeichnet, dass der Fermentierer des festen Substrats oberhalb des Behälters für die Fermentation des flüssigen Substrats und/oder des in Lösung befindlichen Substrats angeordnet ist, damit ein Abfliessen der Brühe, die von dem oberen Fermentierer stammt, zu dem unteren Fermentierer durch Schwerkraft möglich ist.

7. Einrichtung nach einem der Ansprüche 1 bis 6, dadurch gekennzeichnet, dass das Gas, das in den Fermentierern erzeugt worden ist, direkt durch Rohre und Leitungen, welche von den Fermentierern ausgehen, abgeführt wird, während eine gewisse Menge dieses Gases, bevor es abgeführt wird, durch die hydraulische Dichtung hindurchgeht, die hinter der Einrichtung zum Einspeisen des festen Substrats vorgesehen ist.

## Claims

1. Installation for the preparation of fuel gases, by anaerobic fermentation, characterised in that it comprises in combination: at least one tank (1) for the treatment of solids or of very concentrated substrates (having a content higher than 16% of dry matter), said tank being provided with perforated partitions (4, 17) ensuring drainage over substantially the height of the tank, holding the solids and allowing the liquids and the gases to circulate; at least one tank (2) for the treatment of dilute substrates (whose content of dry matter is less than 10%) equipped with a heating coil (13); a device for introducing solid substrates (5) provided with a chopper (7), with an inlet tube (8) for juice recirculated from the fermentation tank of the liquid substrate and a propelling member (25) comprising a propelling helix (24), pump or the like, terminated possibly by a distributing member (11); an apparatus for introducing liquid substrates (6); a collecting device (14, 15) for the fermentation residue coming from the tank or tanks for fermenting solid substrates; connecting piping (16a, 16b) between the fermentation tanks of solid substrate and the fermentation tanks of liquid substrate, said piping connecting the fermentation tanks at different levels; a collecter pipe (12) for gases produced by fermentation.

2. Installation according to claim 1, characterised in that the various elements forming the introduction device for the solid matter are arranged so as to form two successive front and rear hydraulic seals between the outer atmosphere and the inner space of the solid substrate fermentation tank.

3. Installation according to claim 1, characterised in that the perforated partition with which the solid substrate fermentation tank is provided has the form of a basket having the shape of said tank.

4. Installation according to claim 1, characterised in that the perforated partition with which the fermentation tank of the solid substrate is provided is arranged so as to separate the tank into several zones containing solid substrate and several zones for the flow of the liquids and of the gases.

5. Installation according to any one of claims 1 to 4, characterised in that the collecting device for the fermentation residue coming from the fermentation tank of the solid substrate is composed of a set of grids equipped with an easily manoeuvrable mechanism for the extraction of the fermented substrate, and with an evacuation hopper (15) provided with an extraction member constituting a hydraulic seal for isolating the inner space of the tank.

6. Installation according to any one of claims 1 to 5, characterised in that the fermenter of the solid substrate is situated above the fermentation tank of the liquid and/or dissolved substrate, in order to permit the flow by gravity of the juice coming from the upper fermenter to the lower fermenter.

7. Installation according to any one of claims 1 to 6, characterised in that the gas produced in the fermenters is collected directly by piping and ducts coming from the fermenters, whilst a certain amount of this gas passes before being collected, through the rear hydraulic seal of the device for supplying solid substrate.

Fig.1

0 038 759

9

0 038 759

Fig. 2

Fig. 3